# EUROPEAN PATENT APPLICATION

(11) **EP 3 434 783 A1**
(43) Date of publication of application: **30.01.2019**
(21) Application number: 17774837.3
(22) Date of filing: 25.03.2017
(51) Int. Cl.: C12Q 1/68, A23L 33/18, A61K 8/46, A61K 31/185, A61P 43/00, A61Q 19/08, C12N 15/09, G01N 33/68

(54) **MITOCHONDRIAL BIOMARKER REFLECTING AGING**

(30) Priority: 26.03.2016 JP 2016062910
(71) Applicant: Kawasaki Gakuen Educational Foundation, Kurashiki-shi, Okayama 701-0192 (JP)
(72) Inventor: SUNADA, Yoshihide, Kurashiki-shi Okayama 701-0192 (JP); OHSAWA, Yutaka, Kurashiki-shi Okayama 701-0192 (JP); NISHIMATSU, Shin-ichiro, Kurashiki-shi Okayama 701-0192 (JP)
(74) Representative: J A Kemp
(86) International application number: PCT/JP2017/012205
(87) International publication number: WO 2017/170300

(57) **Abstract**

The present invention provides a method for measuring senescence of a test subject, including a step of measuring a level of taurine modification of mitochondrial tRNA^{Leu(UUR)} in a biological sample isolated from a test subject, by a reverse transcription reaction from a primer with the tRNA^{Leu(UUR)} as a template. In addition, the present invention also provides a senescence determining drug containing a primer containing a base sequence consisting of 10 - 25 bases and complementary to the mitochondrial tRNA^{Leu(UUR)}. Furthermore, the present invention also provides a senescence determining kit containing a primer containing a base sequence consisting of 10 - 25 bases and complementary to the mitochondrial tRNA^{Leu(UUR)} and a reverse transcriptase. Moreover, the present invention also provides an agent for improving a taurine modification rate of mitochondrial tRNA^{Leu(UUR)}, containing taurine as an active ingredient.

## Description

### [Technical Field]

The present invention relates to a mitochondrial biomarker reflecting senescence, and to a field that visualizes senescence and provides an index of health expectancy.

### [Background Art]

Mitochondrion is an intracellular organella in charge of aerobic metabolism of somatic cells, and produces ATP that becomes energy by hydrogen transfer system and oxidative phosphorylation. It is considered that this mitochondrial function is obstructed not only by mitochondrial diseases caused by mutation of mitochondrial DNA having a replication mechanism independent of genome but also senescence. Therefore, early diagnosis of mitochondrial dysfunction and impairment evaluation not only play an important role in the selection of therapeutic strategy for and judgment of prognosis of patients with mitochondrial diseases but also are attracting attention as a senescence marker.

MELAS is a mitochondrial disease with the highest frequency, exhibiting diverse and characteristic symptoms represented by myopathy, encephalopathy, lactic acidosis and stroke-like episode. Its life expectancy from diagnosis is 5-10 years, and it is a refractory rare disease without any medicine and applicable to insurance worldwide. It progresses with repeated stroke-like attacks, and a therapy for suppressing recurrence thereof has been desired to be provided as soon as possible. MELAS is developed by single base substitution (A3243G, T3271C, G3244A, T3258C, T3291C) in the tRNA^{Leu(UUR)} gene coding region of mitochondrial DNA. Its basic pathology has long been unknown. Ohta et al. of Nippon Medical School found a unique basic pathology that taurine modification of the first letter of anticodon, which is normally observed, is lost and translational defect is induced in MELAS mutated tRNA^{Leu(UUR)}, and proposed a novel disease concept of "tRNA post-transcriptional modification abnormality disease" first in the world (non-patent document 1). The present inventors jointly studied with Ohta et al. and reported that high-dose administration of taurine improved mitochondrial dysfunction of MELAS model cells and frequent stroke-like attacks have completely been suppressed for not less than 10 years in two patients (non-patent document 2). They performed Multicenter, Open, Phase 3, Doctor-initiated clinical trial to verify the efficacy of the MELAS stroke-like attack suppressive effect of a GCP test drug taurine (Health Labour Sciences Research Grant: H24-26 refractory and the like (refractory)-general-068), and achieved complete suppression of the key evaluation item, MELAS stroke-like attack, in 60% of the patients.

A compendia relating to the role of tRNA modification in human diseases has been published (non-patent document 3), and the measurement method (non-patent document 4) and compendia relating to the lack of taurine modification of mitochondrial tRNA in human mitochondrial diseases have also been published (non-patent document 5). In addition, some patent applications directed to the diagnosis or treatment taking note of mitochondrial tRNA have been filed.
1) A method for diagnosing type 2 diabetes by chemical modification of tRNA including mitochondrial tRNA, specifically, by measuring the thiomethyl modification rate of tRNA encoding lysine, is known (patent document 1).
2) A method for improving health condition of aging animals by preventing or repairing changes in metabolism associated with aging, particularly changes leading to mitochondrial functional disorder, is known (patent document 2). Glutathione is a major intracellular antioxidant system that prevents oxidative damage of mitochondrial DNA (mtDNA). Oxidation of glutathione itself proceeds with aging and the antioxidant function decreases. It is described relating thereto that the oxidative damage of mtDNA can be prevented by supplying a thiol compound such as taurine and the like. However, the relationship between taurine modification rate of tRNA^{Leu(UUR)} and senescence is not described.
3) A therapeutic drug for a mitochondrial disease containing taurine, taurine chloramine, taurine precursor and the like as an active ingredient is known (patent document 3). However, it is not disclosed or suggested that the taurine modification rate of normal mitochondrial tRNA^{Leu(UUR)} decreases due to aging and senescence (decline of biological function) due to genetic factors, lifestyle and environmental factors and the like and that the decrease is improved by administering taurine.

### [Document List]

### [Patent documents]

patent document 1: WO 2014-136870
patent document 2: National Publication of International Patent Application No. 2004-519241
patent document 3: JP-A-2003-48829

### [non-patent document]

non-patent document 1: Yasukawa T, Suzuki T, Ueda T, Ohta S, Watanabe K. J Biol Chem. 2000; 275(6):4251-4257
non-patent document 2: Rikimaru M, Ohsawa Y, Wolf AM, Nishimaki K, Ichimiya H, Kamimura N, Nishimatsu S, Ohta S, Sunada Y. Intern Med. 2012; 51(24):3351-3357.
non-patent document 3: Adrian Gabriel Torres, Eduard Batlle, Lluis Ribas de Poulana, Trends in Molecular Medicine 2014; 20 (6) : 306-314
non-patent document 4: Kirino Y, Goto Y, Campos Y, Arenas J, SuzukiT. Proc Natl Acad Sci U S A.2005; 102(20):7127-7132. non-patent document 5: Tsutomu Suzuki, Asuteka Nagao, and Takeo Suzuki, WIREs RNA 2011; 2:376-386

### [SUMMARY OF THE INVENTION]

### [Problems to be Solved by the Invention]

A first object of the present invention is to provide a novel biomarker leading to an early detection and impairment severity evaluation of mitochondrial dysfunction associated with senescence.

A second object of the present invention is to provide an agent for improving a decrease in the taurine modification rate of mitochondrial tRNA^{Leu(UUR)}, which induces decline in the mitochondrial function and is associated with senescence.

### [Means of Solving the Problems]

Lactate value of blood and cerebrospinal fluid, and the ratio of lactic acid value to pyruvic acid value that have been clinically used heretofore as mitochondrial disease markers rise due to conditions in which anaerobic metabolism is promoted by mitochondrial disease. They also rise due to glycolytic metabolism promoted by tissue hypoxia, that is, various pathologies such as shock, respiratory failure, disseminated intravascular coagulation syndrome and the like. Thus, these markers have low specificity (Kraut JA, Madias NE. Lactic acidosis. N Engl J Med. 2015; 372(11):1078-1079). The present inventors have measured the taurine modification rate of mitochondrial tRNA^{Leu(UUR)} of leukocyte samples of the test subjects and intensively studied usefulness of the mitochondrial disease marker. Specifically, they observed not only a decrease in the taurine modification rate of mutated mitochondrial tRNA^{Leu(UUR)} but also a decrease in the taurine modification rate of normal mitochondrial tRNA^{Leu(UUR)} in mitochondrial disease patients, and unexpectedly found that the taurine modification rate of normal mitochondrial tRNA^{Leu(UUR)} could be an index of senescence. Then they conceived the index can be applied to healthy human as well as mitochondrial disease patients, which resulted in the completion of the present invention. Furthermore, the present inventors have found that the administration of a large amount of taurine not only increases the taurine modification rate of mutated mitochondrial tRNA^{Leu(UUR)}, but also increases the taurine modification rate of normal mitochondrial tRNA^{Leu(UUR)} in mitochondrial disease patients, which resulted in the completion of the present invention.

The present invention provides the following.
[1] A method for measuring senescence of a test subject, comprising a step of measuring a level of taurine modification of mitochondrial tRNA^{Leu(UUR)} in a biological sample isolated from a test subject, by a reverse transcription reaction from a primer with the tRNA^{Leu(UUR)} as a template.
[2] The method of [1], wherein the primer is an oligonucleotide 10 - 25 bases in length and complementary to the template, and a difference in primer elongation products is detected based on the presence or absence of taurine modification.
[3] The method of [1] or [2], wherein the primer is at least one kind comprising a base sequence selected from the group consisting of base sequences shown in SEQ ID NOs: 3, 4 and 5.
[4] The method of any of [1] to [3], further comprising a step of calculating a quantitative ratio of a primer elongation product from tRNA^{Leu(UUR)} with taurine modification and a primer elongation product from tRNA^{Leu(UUR)} without taurine modification.
[5] The method of any of [1] to [4], wherein a total taurine modification rate represented by the following formula (1): $total taurine modification rate = τ {m}^{5} U / \left(U + τ {m}^{5} U\right) x 100 \left(%\right)$ wherein τm⁵U is an amount of primer elongation product from tRNA^{Leu(UUR)} with taurine modification and U is an amount of a primer elongation product from tRNA^{Leu(UUR)} without taurine modification, is an index of senescence.
[6] The method of [5], wherein the test subject is suspected to have a mutated mitochondrial DNA having a single base substitution selected from the group consisting of A3243G, T3271C, G3244A, T3258C and T3291C in the tRNA^{Leu(UUR)} gene coding region, and a normal taurine modification rate determined by the following formula (2): $normal taurine modification rate = total taurine modification rate / \left(100 - mtDNA point mutation rate\right) / 100 \left(%\right)$ (in the above-mentioned formula, the mtDNA point mutation rate is the highest point mutation rate selected from the group consisting of point mutations at 14-position A (A3243G), 15-position G (G3244A), 29-position T (T3258C), 42-position T (T3271C) and 62-position T (T3291C) in a DNA encoding tRNA^{Leu(UUR)} shown in SEQ ID NO: 1) successively from the aforementioned formula (1) is the index of senescence.
[7] A senescence determining drug comprising a primer comprising a base sequence consisting of 10 - 25 bases and complementary to the mitochondrial tRNA^{Leu(UUR)}.
[8] The drug of [7], wherein the primer is at least one kind comprising a base sequence selected from the group consisting of the base sequences shown in SEQ ID NOs: 3, 4 and 5.
[9] A senescence determination kit comprising a primer comprising a base sequence consisting of 10 - 25 bases and complementary to the mitochondrial tRNA^{Leu(UUR)} and a reverse transcriptase.
[10] The kit of [9], wherein the primer is at least one kind comprising a base sequence selected from the group consisting of the base sequences shown in SEQ ID NOs: 3, 4 and 5.
[11] An agent for improving a taurine modification rate of mitochondrial tRNA^{Leu(UUR)}, comprising taurine as an active ingredient.
[12] The agent of [11], wherein the mitochondrial tRNA^{Leu(UUR)} is normal mitochondrial tRNA^{Leu(UUR)}.
[13] The agent of [11], wherein the improvement of taurine modification rate is improvement of a total taurine modification rate determined from the following formula (1): $total taurine modification rate = τ {m}^{5} U / \left(U + τ {m}^{5} U\right) x 100 \left(%\right)$ wherein τm⁵U is an amount of a primer elongation product from tRNA^{Leu(UUR)} with taurine modification and U is an amount of a primer elongation product from tRNA^{Leu(UUR)} without taurine modification, and/or a normal taurine modification rate determined from the following formula (2): $normal taurine modification rate = total taurine modification rate / \left(100 - mtDNA point mutation rate\right) / 100 \left(%\right)$ (in the above-mentioned formula, the mtDNA point mutation rate is the highest point mutation rate selected from the group consisting of point mutations at 14-position A (A3243G), 15-position G (G3244A), 29-position T (T3258C), 42-position T (T3271C) and 62-position T (T3291C) in a DNA encoding tRNA^{Leu(UUR)} shown in SEQ ID NO: 1).
[14] The agent of [11] or [12], which is a medicament or cosmetic.
[15] The agent of [11] or [12], which is a food with health claims or food additive.
[16] The agent of any of [11] to [15], which is for improving a taurine modification rate of mitochondrial tRNA^{Leu(UUR)} of a patient who developed a mitochondrial disease and/or a subject having an onset risk.
[17] The agent of [16], wherein the mitochondrial disease is caused by a mutation of a gene deficient in taurine modification of mitochondrial tRNA^{Leu(UUR)}.
[18] The agent of [17], wherein the mutation of a gene deficient in taurine modification of mitochondrial tRNA^{Leu(UUR)} is a point mutation selected from the group consisting of the 14-position A (A3243G), the 15-position G (G3244A), the 29-position T (T3258C), the 42-position T (T3271C) and the 62-position T (T3291C) in a DNA encoding tRNA^{Leu(UUR)} shown in SEQ ID NO: 1).
[19] The agent of any of [16] to [18], wherein the mitochondrial disease is MELAS or diabetes.

### [Effect of the Invention]

The present inventors have found that the taurine modification rate of normal tRNA^{Leu(UUR)}, among the mitochondrial tRNAs, correlates to aging, unlike the blood lactic acid value, ratio of blood lactic acid value to pyruvic acid value, cerebrospinal fluid lactic acid value, cerebrospinal fluid lactic acid value and pyruvic acid value, which are conventional markers of mitochondrial diseases. Therefore, the taurine modification rate of normal tRNA^{Leu(UUR)} is a universal marker capable of grasping the state of physiological function of not only mitochondrial disease patients but also healthy individuals.

### [Brief Description of the Drawings]

Fig. 1 shows the outline of a measurement method of the taurine modification of mitochondrial tRNA^{Leu(UUR)} by the primer elongation method.
Fig. 2 shows correlations between age, and total taurine modification rate or taurine modification rate of normal mitochondrial tRNA^{Leu(UUR)} of MELAS patients.
Fig. 3 shows relationships between blood lactic acid value, and taurine modification rate of normal mitochondrial tRNA^{Leu(UUR)} or age of MELAS patients.
Fig. 4 shows relationships between blood lactic acid/pyruvic acid ratio, and taurine modification rate of normal mitochondrial tRNA^{Leu(UUR)} or age of MELAS patients.
Fig. 5 shows relationships between cerebrospinal fluid lactic acid/pyruvic acid ratio, and taurine modification rate of normal mitochondrial tRNA^{Leu(UUR)} or age of MELAS patients.
Fig. 6 shows relationships between cerebrospinal fluid lactic acid value, and taurine modification rate of normal mitochondrial tRNA^{Leu(UUR)} or age of MELAS patients.
Fig. 7 shows changes in the total taurine modification rate due to taurine administration to MELAS patients, and improvement of the taurine modification rate of normal tRNA^{Leu(UUR)}.
Fig. 8 shows taurine modification rates of mitochondrial tRNA^{Leu(UUR)} of healthy young people (10's) and healthy elderly people (80's).

### [Description of Embodiments]

In the present specification, indication of amino acid, (poly)peptide, (poly)nucleotide and the like using abbreviations follows the provision of IUPAC-IUB [IUPAC-IUB Communication on Biological Nomenclature, Eur. J. Biochem., 138: 9 (1984)], "Guideline for preparation of minute descriptions of compounds including base sequence or amino acid sequence" (ed. Japan Patent Office), and conventional symbols used in the pertinent field.

### Definition

In the present invention, "senescence" means that biological functions decrease due to aging and other internal or external factors such as genetic factors, lifestyle and environmental factors and the like. Aging is physical progress of time from birth to death and is synonymous with chronological age. Such other internal factors include, for example, those relating to cellular senescence such as mitochondria function, peroxide radical concentration, telomere length and the like, and the like. Such other external factors include, for example, individual amount of motor activity, smoking or nonsmoking, dietary habits, nutritional status and the like. The biological function includes, for example, muscular strength, nerve conduction velocity, vital capacity, disease resistance and the like. Senescence due to aging generally starts after reaching the reproductive age, and starts from 20 to 30 years old in humans, though subject to individual differences. Senescence is considered to involve environmental factors and genetic predisposition that are intertwined complicatedly. The speed of such functional deterioration is not the same for all humans, and cannot necessarily be defined by the factor of aging alone. Conventionally, therefore, there is no clear index of senescence. The index of senescence provided by the present invention can be used as the index also from the aspect of health expectancy.

The "senescence" in the present invention includes not only external changes such as reduction of height and body weight, change of skin and hair and the like, and what is called age-related decline in physical functions such as decline of motility functions, decline in sensory functions, decline in physiological functions and the like, but also senescence of cells constituting the living body (cellular senescence), and further, senescence of mitochondria that plays an important role in respiration and energy metabolism. When the taurine modification rate of mitochondrial tRNA^{Leu(UUR)} decreases due to senescence, the UUG codon-specific translation ability naturally becomes rate-limiting and the synthesis of proteins containing UUG codons decreases. This in turn decreases mitochondrial functions, and clearly decreases functions of the organs and the like that constitute the living body. When the decline of the modification rate proceeds further, for example, severe symptoms of dyspnea and skeletal muscle attenuation found in the elderly people are also considered to be developed in some cases. Therefore, it is needless to say that taurine modification rate of normal mitochondrial tRNA^{Leu(UUR)} can be used as an index of senescence and functional decline of mitochondria themselves, and is also useful as an index of the function of a living body, organ, tissue, cell or intracellular organelle, in particular, an index of senescence and functional decline of living body, organ, tissue, cell, or organelle with high energy demand.

In addition, the above-mentioned index is also useful for selection of subjects of taurine therapy currently under development for mitochondrial disease patients, use for companion diagnosis to be used for determination of the treatment effect thereof and assistance therefor.

The health expectancy here means a period defined by the Ministry of Health, Labor and Welfare, during which one can live without limitation on everyday life due to health problems. Each country including the World Health Organization aims to extend health expectancy, and it is desired to ultimately make the difference between the health expectancy and average life span close to zero.

In the present invention, mitochondrial tRNA^{Leu(UUR)} is a tRNA that is transcribed from mitochondrial DNA and recognizes the codon UUR (R is A or G) of leucine. In mitochondrial tRNA^{Leu(UUR)}, the bases at the positions 17, 20 and 47 increase or decrease depending on the animal species, and therefore, the position of the nucleotide (UAA) corresponding to the anticodon is unified to be the 34-position to 36-position. DNA encoding human mitochondrial tRNA^{Leu(UUR)} is registered in NCBI and published under GenBank accession No. AB026838 (version AB026838.1) (SEQ ID NO: 1). The mitochondrial tRNA^{Leu(UUR)} transcribed from the DNA shown in SEQ ID NO: 1 is shown in SEQ ID NO: 2. The 36th (34-position according to the above-mentioned definition) uridine (U) of SEQ ID NO: 2 may or may not be taurine modified.

In the present specification, "mitochondrial tRNA^{Leu(UUR)}" is sometimes to be simply abbreviated as "tRNA^{Leu(UUR)}".

Various mutations have been found in tRNA^{Leu(UUR)}. In the present invention, the measurement target includes not only tRNA^{Leu(UUR)} shown in SEQ ID NO: 2 but also mutated tRNA^{Leu(UUR)} known to the present and mutated tRNA^{Leu(UUR)} to be found in the future. Specific examples of the mutated tRNA^{Leu(UUR)} include, in the DNA encoding tRNA^{Leu(UUR)} shown in SEQ ID NO: 1, point mutations at the 14-position A (A3243G), 15-position G (G3244A), 29-position T (T3258C), 42-position T (T3271C), 62-position T (T3291C): these mutations cause lack of taurine modification and are found in MELAS patients; point mutations at the 13-position G (A3242A), 21-position T (T3250C), 25-position C (C3254T), 51-position A (A3280G): these mutations have no effect on taurine modification and are found in mitochondrial diseases other than MELAS; and the like. For other mutations, MITOMAP (www.mitomap.org/MITOMAP) can be referred to.

In the present invention, mutated tRNA^{Leu(UUR)} refers to tRNA^{Leu(UUR)} transcribed from mtDNA having mutation causing deficiency of taurine modification and does not include tRNA^{Leu(UUR)} having mutation with no effect on taurine modification.

In addition, normal tRNA^{Leu(UUR)} refers to tRNA^{Leu(UUR)} other than the above-mentioned mutated tRNA^{Leu(UUR)} . In human, tRNA^{Leu(UUR)} having the same base sequence as tRNA^{Leu(UUR)} shown in SEQ ID NO: 2 and tRNA^{Leu(UUR)} having a different base sequence from tRNA^{Leu(UUR)} shown in SEQ ID NO: 2 in which the difference in the base sequence does not influence the taurine modification of tRNA^{Leu(UUR)} are included. Those lacking the function of normal tRNA^{Leu(UUR)} are not included irrespective of the presence or absence of taurine modification.

The above-mentioned 14-position A, 15-position G, 29-position T, 42-position T and 62-position T are also conserved in gorilla, bovine, swine, dog, cat and the like besides human. In rat and mouse, they are similarly conserved except that the 42-position is C. Therefore, the mutation causing deficient taurine modification of tRNA^{Leu(UUR)} is not limited to human and the below-mentioned mammals such as primates, pets, domestic animals, experiment animals and the like can also be the targets.

In the present invention, taurine modification of mitochondrial tRNA^{Leu(UUR)} refers to post-transcriptional modification in which taurinomethyl group is bonded to the 5-position of the uracil base at the first letter of anticodon (UAA) of tRNA^{Leu(UUR)} (wobble position at the 34-position according to the above-mentioned definition). Uridine which is taurine-modified in this way is sometimes shown with abbreviation of τm⁵U.

### Mitochondrial DNA (mtDNA) and heteroplasmy

Some hundreds of mitochondria are present per cell, and each one of mitochondria contains 5 to 10 mitochondrial DNAs (mtDNAs). When constituted of a single mtDNA, it is called "homoplasmy", and when normal and mutant mtDNAs are mixed, it is called "heteroplasmy". When the ratio of mutated mtDNA (heteroplasmy degree) is high, mitochondrial respiration function becomes abnormal and a mitochondrial disease is developed. The degree of heteroplasmy varies depending on the tissues such as skeletal muscle, blood vessel, skin and the like, or cells. It was recently reported that tissues and cells of healthy human also contain a trace amount of mutated mtDNA (Brendan A.I. Payne et al.: Universal heteroplasmy of human mitochondrial DNA. Hum Mol Gent 22: 384 - 390 (2013)). In the present invention, therefore, the degree of taurine modification of mitochondrial tRNA^{Leu(UUR)} is defined as follows and the test subjects are divided into those having substantially normal mtDNA (healthy human) and those containing mutated mtDNA at a certain level (e.g., MELAS patients, diabetes patients), and taurine modification rate of tRNA^{Leu(UUR)} is measured.

### Taurine modification rate of mitochondrial disease patients

Mitochondria of MELAS patients' cells are heteroplasmy and contain mutated mtDNA and normal mtDNA. Therefore, even if the first uracil in the anticodon of tRNA^{Leu(UUR)} transcribed from mutated mtDNA lacks taurine modification, tRNA^{Leu(UUR)} transcribed from normal mtDNA is assumed to be taurine modified.

The taurine modification rate of tRNA^{Leu(UUR)} transcribed from normal mtDNA contained in the cell derived from a test subject containing mutated mtDNA such as MELAS patients is defined as "normal taurine modification rate" and can be determined from the following formula (2) based on the "total taurine modification rate" determined from the following formula (1).

In the present invention, the total taurine modification rate is a value obtained by dividing taurine modified tRNA^{Leu(UUR)} in mitochondrial tRNA^{Leu(UUR)} in a sample derived from a test subject by a total sum of taurine modified tRNA^{Leu(UUR)} and taurine non-modified tRNA^{Leu(UUR)}.

The test subjects here are mammals including human. Examples of the mammal include rodents such as mouse, rat, hamster, guinea pig and the like, experiment animals such as rabbit and the like, domestic animals such as swine, bovine, goat, horse, sheep, mink and the like, pets such as dog, cat and the like, primates such as human, monkey, Macaca fascicularis, Macaca mulatta, marmoset, orangutan, chimpanzee, gorilla and the like, and the like. The sample derived from a test subject is not particularly limited as long as it can be collected from mammals, preferably human, and body fluid samples such as blood, lymph fluid, urine and the like, and biopsy tissues such as hair, buccal mucosa, stomach, large intestine, lung, liver, brain and the like can be mentioned. Preferred is a blood sample, and leukocyte in a blood sample is more preferable.

The total taurine modification rate is a value obtained by indirectly determining the amounts of taurine modified tRNA^{Leu(UUR)} and taurine non-modified tRNA^{Leu(UUR)} by measuring the amount of a primer elongation product by a reverse transcription reaction (primer elongation method) using a primer, and assigning them in the following formula (1): $total taurine modification rate = τ {m}^{5} U / \left(U + τ {m}^{5} U\right) x 100 \left(%\right)$ wherein τm⁵U is an amount of primer elongation product from tRNA^{Leu(UUR)} with taurine modification and U is an amount of a primer elongation product from tRNA^{Leu(UUR)} without taurine modification.

When the test subject is suspected to have a mutated mitochondrial DNA having a single base substitution selected from the group consisting of A3243G, T3271C, G3244A, T3258C and T3291C in the tRNA^{Leu(UUR)} gene coding region, a normal taurine modification rate is determined by the following formula (2): $normal taurine modification rate = total taurine modification rate / \left(100 - mtDNA point mutation rate\right) / 100 \left(%\right)$ (in the above-mentioned formula, the mtDNA point mutation rate is the highest point mutation rate selected from the group consisting of point mutations at 14-position A (A3243G), 15-position G (G3244A), 29-position T (T3258C), 42-position T (T3271C) and 62-position T (T3291C) in a DNA encoding tRNA^{Leu(UUR)} shown in SEQ ID NO: 1) successively from the aforementioned formula (1) and desirably used as the index of senescence.

For the analysis of point mutation rate of mtDNA, DNA sequencing method, PCR-RFLP method, PCR-SSCP method, denaturing high-performance liquid chromatography (DHPLC) method, BiPlex Invader method, SnaP shot method, high-resolution melt (HRM) profiling method, temporal temperature gradient gel electrophoresis (TTGE) method, a method using a next-generation sequencer currently under development and the like can be mentioned.

The total taurine modification rate is theoretically within the range of 0 - 100%. Considering the pathology of MELAS patients, severe symptoms may be developed when the total taurine modification rate decreases. On the other hand, when the normal taurine modification rate is calculated using the aforementioned calculation formula (2), it is almost the same as the total taurine modification rate because the mtDNA point mutation rate is almost 0% in healthy human. However, the normal taurine modification rate of mitochondrial disease patients may exceed 100% by calculation due to mtDNA point mutation rate.

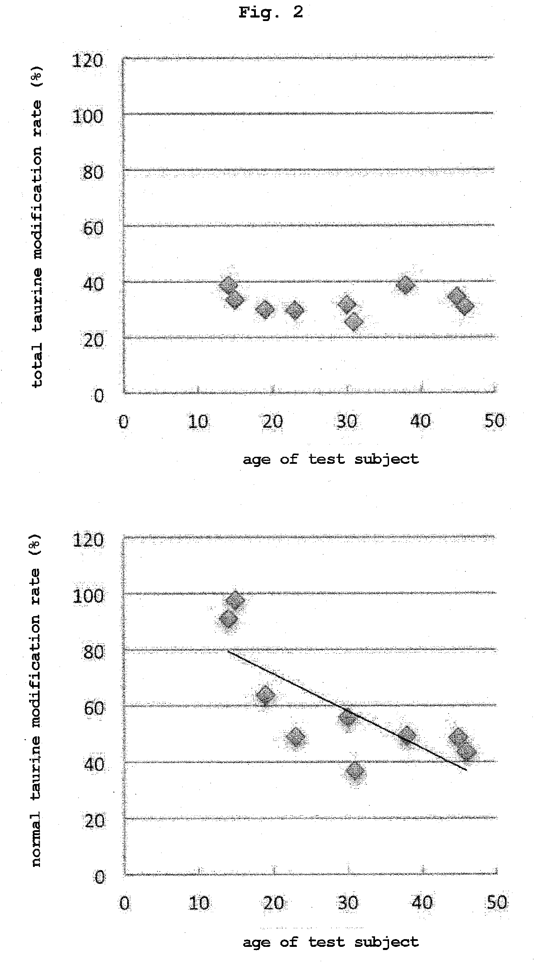

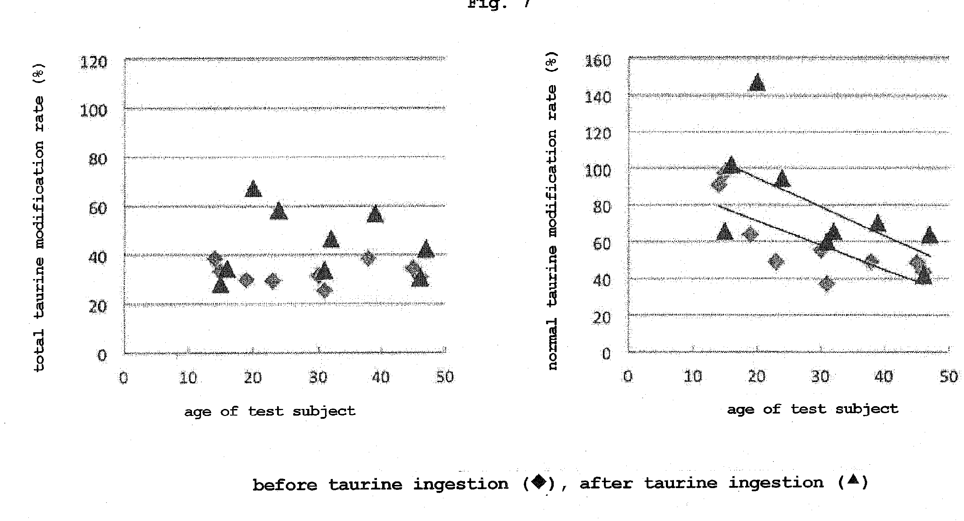

In the doctor-initiated clinical trial, the normal taurine modification rate of tRNA^{Leu(UUR)} was plotted on the vertical axis and the age of the test subject on the horizontal axis. Thus, it was found that the normal taurine modification rate decreased as the age of the test subject increased (Fig. 2). In addition, it was clarified that taurine ingestion by MELAS patients restores normal taurine modification rate and improves taurine modification of tRNA^{Leu(UUR)} derived from mutant mtDNA (Fig. 7, Table 1). It is expected that administration of an effective amount of taurine delays the progression of senescence.

It can be judged that a lower normal taurine modification rate indicates further progression of senescence. That is, the normal taurine modification rate can be used to indicate the measure of senescence as "Taurine Age". In healthy human, the total taurine modification rate is close to normal taurine modification rate, and the total taurine modification rate can be conveniently used to indicate the senescence degree as "taurine age".

### Measurement method of senescence

### (1) a step of measuring a level of taurine modification of mitochondrial tRNA^{Leu(UUR)} in a biological sample isolated from a test subject, by a reverse transcription reaction from a primer with the tRNA^{Leu(UUR)} as a template

Non-limitative examples of the biological sample to be the measurement target include biopsy tissues of hair, buccal mucosa, stomach, large intestine, lung, liver, brain and the like, and body fluid samples of blood, lymph fluid, urine and the like. Blood is preferable since it is easily collected, and leukocyte obtained from blood is more preferable. In the following, the measurement method of senescence is explained by taking leukocyte as an example. Other biological samples can also be measured similarly.

The leukocyte to be measured can be obtained by collecting blood from a test subject and recovering the leukocyte fraction by a conventional method. For this purpose, commercially available reagents can be preferably used. Examples of the commercially available reagent include Lymphoprep (registered trade mark), Ficoll (registered trade mark)-Paque and the like. The leukocyte fraction is obtained by performing density gradient centrifugation according to the instructions of the manufacturer of the reagent and separating the blood cell layer including the lymphocytes and monocytes (peripheral blood mononuclear cell: PBMC).

The test subject is typically human, and may be a mammal excluding human. Examples of the mammal excluding human include rodents such as mouse, rat, hamster, guinea pig and the like, experiment animals such as rabbit and the like, domestic animals such as swine, bovine, goat, horse, sheep, mink and the like, pets such as dog, cat and the like, primates such as monkey, Macaca fascicularis, Macaca mulatta, marmoset, orangutan, chimpanzee, gorilla and the like, and the like.

A sample containing RNA is prepared from a leukocyte fraction derived from the test subject by a conventional method. As a method of extracting RNA from cells, it is desirable to adopt a method of simultaneously performing cell disruption and inactivation of RNase. Cells are disrupted and solubilized with a solubilizing agent, proteins are removed with a denaturing agent, gene is precipitated with ethanol or the like, and RNA can be prepared from leukocytes. Commercially available extraction kits can be preferably used for this operation. For example, Isogen (manufactured by Nippon Gene), Trizol (manufactured by Ambion) and the like can be mentioned.

The obtained RNA can be subjected to a reverse transcription reaction without further purification. To use tRNA^{Leu(UUR)} contained in the obtained RNA as a template, a primer that specifically hybridizes to the tRNA^{Leu(UUR)} is used. In the present invention, the primer is an oligonucleotide complementary to the template. As the oligonucleotide, DNA, RNA, hybrid of DNA and RNA may be used. From the aspect of operability, DNA is preferable. The nucleotide constituting the oligonucleotide may be a natural nucleotide or an artificial nucleotide.

The length of the primer is not particularly limited as long as it can retain a function capable of specifically hybridizing to tRNA^{Leu(UUR)}. It is 8 - 30 bases in length, preferably 10 - 25 bases in length, more preferably 10 - 22 bases in length.

The specific base sequence of the primer is designed such that the length of the primer elongation product differs depending on the presence or absence of taurine modification of tRNA^{Leu(UUR)}, or the length of the primer elongation product is different from that of the base sequence. A preferable base sequence is a DNA comprising the base sequence shown in the following:
5'-acctctgactgtaaag-3' (SEQ ID NO: 3)
which is designed to hybridize from 3' to 5' orientation (56-position to 41-position) of tRNA^{Leu(UUR)} shown in SEQ ID NO: 2.

In the primer, within the range of the object of the present invention, one or more (e.g., 2) bases may be added to the 5'-terminal and/or 3'-terminal and one or more (e.g., 2) bases may be deleted from the 5'-terminal and/or 3'-terminal in the base sequence shown in SEQ ID NO: 3.

Furthermore, when the measurement target tRNA^{Leu(UUR)} is assumed to have a mutated base sequence at a position hybridizing with the primer, any base in the base sequence shown in SEQ ID NO: 3 may be substituted with other base such that it becomes complementary to the base sequence of the mutated tRNA^{Leu(UUR)}. To be specific, DNA containing a base sequence of the following case:
5'-acctctgactgtaagg-3' (SEQ ID NO: 4): 3271 mutation, or
5'-acctctgactgcaaag-3' (SEQ ID NO: 5): 3280 mutation can be mentioned. Only one kind of the primers designed in this way may be used or two or more kinds thereof may be used in combination.

The aforementioned primer may be directly or indirectly labeled with a labeling substance. Examples of the labeling substance include fluorescent substance (e.g., FITC, rhodamine, FAM™, VIC™, NED™, PET™, the aforementioned 4 kinds are trade names of Applied Biosystems Inc.), radioactive substance (e.g., ³²P, ³⁵S, ¹⁴C, ³H), enzyme (e.g., alkaliphosphatase, peroxidase), colored particles (e.g., colloidal metal particles, colored latex), biotin and the like. Preferred is a fluorescent substance or a radioactive substance, and the labeling site is desirably the 5'-terminal of the primer.

The reverse transcription reaction can be performed by a known method while referring to Molecular Cloning: A Laboratory Manual, Fourth Edition (2012) and the like. The reverse transcription reaction is performed using reverse transcriptase, the above-mentioned primer and dNTP (nucleotide mixture) in the co-presence of an RNase inhibitor where necessary, and using tRNA^{Leu(UUR)} contained in the obtained RNA as a template.

Reverse transcriptase is commercially available, and a commercially available enzyme kit can be used as long as it is an enzyme capable of performing a reverse transcription reaction using tRNA^{Leu(UUR)} as a template. For example, AMV reverse transcriptase, M-MuLV reverse transcriptase and the like can be mentioned. Mutated reverse transcriptase such as SuperscriptIII (manufactured by Thermo Fisher Scientific Inc.) cannot be used since the reaction is not discontinued at a taurine modification site.

About 1 pg - 1 µg as total RNA is used as the template RNA.

About 0.1 - 1 µg of the primer is used.

As dNTP, a mixture of 4 kinds of nucleotides (e.g., dATP, dGTP, dCTP, dTTP) may be used, or 1 - 3 kinds complementary to the base sequence of the template may also be used. dNTP is generally used at a concentration of about 1-500 µM per nucleotide.

The RNase inhibitor can be used without limitation as long as it can inhibit RNase under the conditions of reverse transcription reaction. Various RNase inhibitors are commercially available and examples of the commercially available product include RNaseOUT (manufactured by Thermo Fisher Scientific Inc.), RNasin (manufactured by Promega) and the like.

The composition of the reverse transcription reaction solution can be appropriately set depending on the reverse transcriptase to be used. In addition, the reaction buffer attached to the commercially available enzyme kit can be diluted and used.

The reverse transcription reaction is divided into an annealing reaction of a template and a primer and a successive elongation reaction from the primer.

In one embodiment of the annealing reaction, for example, conditions for incubation at about 80°C for 1 - 5 min, and standing still at not more than 37°C, generally room temperature (about 25°C) for 10 min - several hours can be mentioned.

In one embodiment of the primer elongation reaction, for example, it is performed at 42°C - 60°C for 15 min - 2 hr and after completion of the reaction, and a heat treatment is performed at 95°C for 1 - 5 min, whereby reverse transcriptase is deactivated.

After completion of the reverse transcription reaction, a difference in the primer elongation product is detected by the presence or absence of taurine modification. For example, a method in which a primer elongation product is developed on agarose gel or polyacrylamide gel by electrophoresis and then detected by a difference in size can be mentioned. In this case, the 5' terminal of the primer is fluorescence labeled or radiolabeled in advance, and after electrophoresis, the signal is taken on an imaging plate and detected using an appropriate detection device.

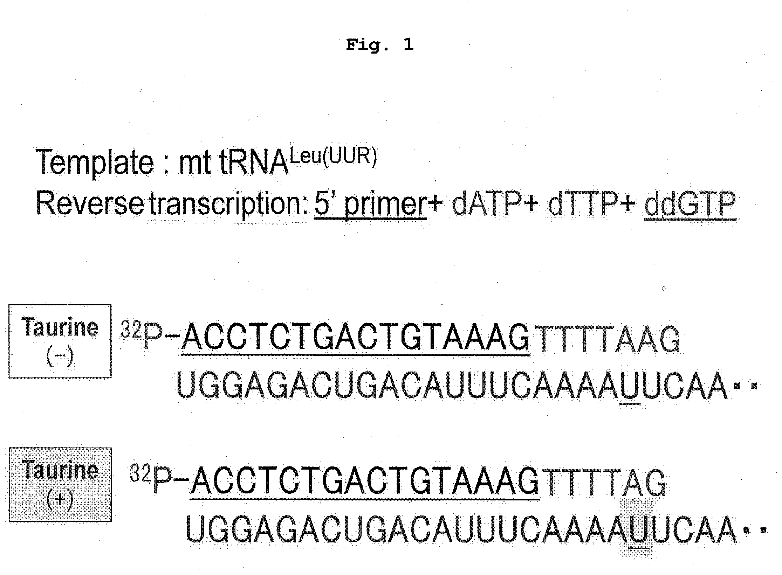

Here, when a reverse transcription reaction is carried out using a primer containing the base sequence shown in SEQ ID NO: 3, τm⁵U modification acts as a barricade against elongation reaction, and the elongation reaction of tRNA^{Leu(UUR)} with taurine modification is discontinued at the 35-position U. In the case of tRNA^{Leu(UUR)} without taurine modification, steric hindrance against elongation reaction is absent and the elongation reaction proceeds, and the elongation reaction can be discontinued at a desired position by the co-presence of dideoxynucleotide (e.g., ddA, ddG, ddC, ddT) in the reaction mixture. For example, when ddG is used, as shown in Fig. 1, the difference between tRNA^{Leu(UUR)} with taurine modification and tRNA^{Leu(UUR)} without taurine modification is shown as a primer elongation product having a length different by one base.

Furthermore, a band separated by electrophoresis may be cut out, the base sequence is determined by a conventional method, and the band may be identified by measuring the difference in the base sequence of the primer elongation product by the presence or absence of taurine modification.

### (2) a step of calculating a quantitative ratio of a primer elongation product from tRNA^{Leu(UUR)} with taurine modification and a primer elongation product from tRNA^{Leu(UUR)} without taurine modification

After electrophoresis of primer elongation product in the aforementioned step, the signal taken into the imaging plate is quantified using densitometry such as BAS 2000 imaging analyzer (manufactured by GE Healthcare) and the like.

The amounts of a primer elongation product from tRNA^{Leu(UUR)} with taurine modification and a primer elongation product from tRNA^{Leu(UUR)} without taurine modification can be expressed in any unit, or may be expressed by a quantitative ratio. These values are assigned in the following formula (1): $total taurine modification rate {=}_{τ} {m}^{5} U / \left(U {+}_{τ} {m}^{5} U\right) x 100 \left(%\right)$ wherein τm⁵U is an amount of primer elongation product from tRNA^{Leu(UUR)} with taurine modification and U is an amount of a primer elongation product from tRNA^{Leu(UUR)} without taurine modification, of the total taurine modification rate is calculated.

When the test subject is suspected to have mutated mtDNA, a normal taurine modification rate is determined by the following formula (2): $normal taurine modification rate = total taurine modification rate / \left(100 - mtDNA point mutation rate\right) / 100 \left(%\right)$ (in the above-mentioned formula, the mtDNA point mutation rate is the highest point mutation rate selected from the group consisting of point mutations at 14-position A (A3243G), 15-position G (G3244A), 29-position T (T3258C), 42-position T (T3271C) and 62-position T (T3291C) in a DNA encoding tRNA^{Leu(UUR)} shown in SEQ ID NO: 1) and desirably used as the index of senescence.

The point mutation rate of mtDNA can be measured using DNA sequencing method, PCR-RFLP method, PCR-SSCP method, denaturing high-performance liquid chromatography (DHPLC) method, BiPlex Invader method, SnaP shot method, high-resolution melt (HRM) profiling method, temporal temperature gradient gel electrophoresis (TTGE) method, a method using a next-generation sequencer currently under development and the like.

For example, in the case of MELAS patients having A3243G, a PCR product amplified using the PCR-RFLP method can be used to quantify the difference in length of the band obtained by the presence or absence of the cleavage site of restriction enzyme ApaI (PCR product with mutation has cleavage site, PCR product without mutation does not have cleavage site), and the point mutation rate of A3243G can be measured. In the case of MELAS patients having I3271C, a PCR product amplified using the PCR-RFLP method can be used to quantify the difference in length of the band obtained by the presence or absence of the cleavage site of restriction enzyme AflII (PCR product with mutation has cleavage site, PCR product without mutation does not have cleavage site), and the point mutation rate of T3271C can be measured.

The thus-obtained total taurine modification rate and normal taurine modification rate can be used as an index of senescence in the present invention. The total taurine modification rate and normal taurine modification rate of healthy subject are almost the same, thus the total taurine modification rate can be used as an index of senescence.

The total taurine modification rate of 100% can be judged to be young from the viewpoint of taurine age.

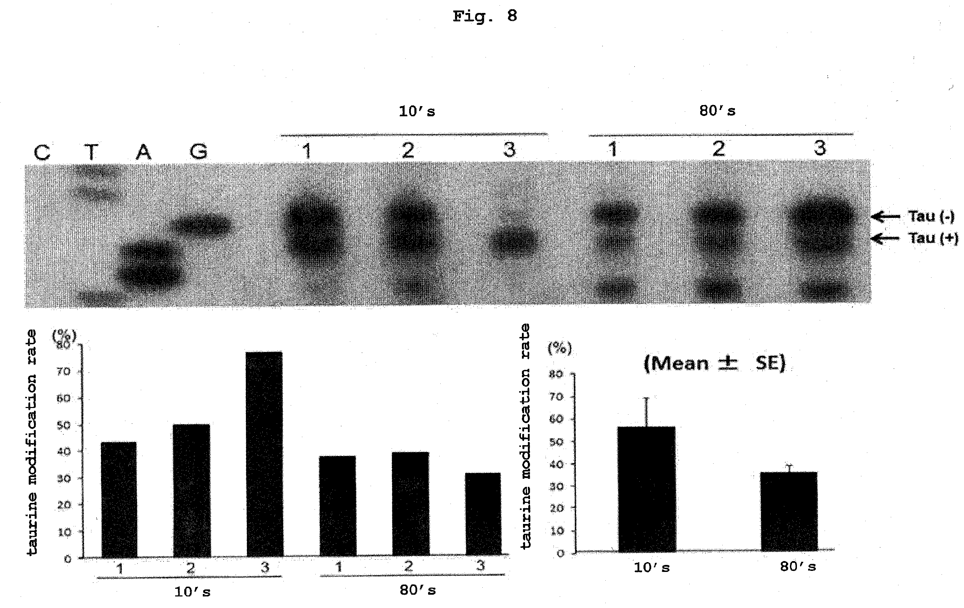

In the analysis results of MELAS patients, it has been newly shown that a decrease in the taurine modification rate of normal mitochondrial tRNA^{Leu(UUR)} has a certain correlation with the chronological age of the patient (Fig. 2). Also in the analysis results of healthy subjects, it has been newly shown that a decrease in the taurine modification rate of normal mitochondrial tRNA^{Leu(UUR)} has a certain correlation with the state of the biological function due to the influence of the chronological age of the patient, genetic factors, lifestyle and environmental factors and the like (Fig. 8). The above-mentioned results indicate that the taurine modification rate of tRNA^{Leu(UUR)} can be used as a biomarker of mitochondrial senescence regardless of healthy subjects or those with mitochondrial diseases. Moreover, it is needless to say that taurine modification rate of normal mitochondrial tRNA^{Leu(UUR)} can be used as an index of senescence and functional decline of mitochondria themselves, and is also useful as an index of the function of a living body, organ, tissue, cell or intracellular organelle, in particular, an index of senescence and functional decline of living body, organ, tissue, cell, or organelle with high energy demand.

In rare cases, taurine modification rates of normal mitochondrial tRNA^{Leu(UUR)} not correlated with chronological age are observed in healthy subjects and MELAS patients. Therefore, the taurine modification rate of normal mitochondrial tRNA^{Leu(UUR)} is not only a mere index of age-related senescence of a test subject but can also be utilized as an index of senescence of the whole physical functions reflecting the health condition of the test subject including the presence or absence of diseases, such as nutritional state, metabolic abnormality and the like, or an index of health expectancy, in particular, health expectancy without any specific cause of death.

In addition, the taurine modification rate of normal mitochondrial tRNA^{Leu(UUR)} is also useful as a biomarker used for determining whether the test subject is normal or has a presymptomatic disease, whether the living conditions of the test subject need improvement, whether a treatment is necessary, and in an assistance method therefor.

The effectiveness of taurine administration has been confirmed not only in MELAS patients but diabetes patients having single base substitution (A3243G) of mitochondrial tRNA^{Leu(UUR)}. It has been shown that the taurine modification rate of normal mitochondrial tRNA^{Leu(UUR)} is also useful as an innovative novel biomarker for selection of subjects of taurine therapy for the mitochondrial disease patients currently under development, application to companion diagnosis used for determination of the therapeutic effects thereof and assistance therefor.

### Senescence determining drug

The primer comprising a base sequence consisting of 10 - 25 bases and complementary to the mitochondrial tRNA^{Leu(UUR)}, which is contained in the senescence determining drug of the present invention, is the same as the primer described in "measurement method of senescence".

The primer may be contained in a freeze-dried state or in a solution state together with a pharmaceutically acceptable carrier.

When the determining drug of the present invention is prepared as a liquid preparation, the pharmaceutically acceptable carrier includes various carriers conventionally used as a preparation material, such as diluent, solvent, solubilizing agent, isotonizing agent, buffering agent and the like.

As the diluent, water, physiological saline and the like can be mentioned.

As the solvent, water, physiological saline, ethanol and the like can be mentioned.

As the solubilizing agent, cyclodextrins and the like can be mentioned.

As the isotonizing agent, inorganic salts such as sodium chloride, potassium chloride and the like, and carbohydrates such as glycerin, mannitol, sorbitol and the like can be mentioned.

As the buffering agent, phosphate buffer, acetate buffer, borate buffer, carbonate buffer, citrate buffer, Tris buffer and the like can be mentioned.

The mixing ratio of these carriers can be appropriately determined by those skilled in the art.

The primer is desirably kept frozen or on ice until immediately before use.

### Senescence determining kit

The senescence determining kit of the present invention contains a primer comprising a base sequence consisting of 10-25 bases and complementary to the mitochondrial tRNA^{Leu(UUR)} and a reverse transcriptase.

The primer comprising a base sequence consisting of 10-25 bases and complementary to the mitochondrial tRNA^{Leu(UUR)}, which is contained in the senescence determining kit of the present invention, is the same as the primer described in "measurement method of senescence".

Preferred embodiments and specific examples of the above-mentioned reverse transcriptase are as described in "measurement method of senescence".

The kit of the present invention may further contain nucleoside triphosphoric acid, buffer for primer elongation reaction, RNase inhibitor.

Nucleoside triphosphoric acid is a substrate incorporated in a primer to constitute a primer elongation product and is generally used as a dNTP mixture. The dNTP mixture is typically composed of dATP, dTTP, dCTP and dGTP.

Where necessary, at least one kind of dideoxynucleotide (ddATP, ddTTP, ddCTP, ddGTP) for discontinuing primer elongation at a desired site may be contained.

Examples of the aforementioned buffer include buffers used for carrying out a general hybridization reaction such as tris buffer, phosphate buffer, veronal buffer, borate buffer, Good's buffer and the like. While the pH thereof is not particularly limited, it is generally preferably 5 - 9.

The aforementioned RNase inhibitor is the same as the RNase inhibitor described in "senescence measurement method".

The kit of the present invention may further contain, in addition to the above-mentioned primer and the like, reaction container, buffer for diluting primer, positive control (e.g., healthy human-derived cybrid cell RNA: see Proc Natl Acad Sci U S A. 2005; 102(20):7127-7132), negative control (e.g., MELAS patient-derived cybrid cell RNA: see Proc Natl Acad Sci USA. 2005; 102(20):7127-7132), manufacturer's instructions describing protocol and the like.

### Agent for improving taurine modification rate of mitochondrial tRNA^{Leu(UUR)}

A decrease in the taurine modification rate of normal mitochondrial tRNA^{Leu(UUR)} due to aging can be improved by taurine administration. The results of Test Example 2 indicate that taurine and a composition containing same are useful for improving taurine modification rate of mitochondrial tRNA^{Leu(UUR)}, preventing a decrease in the taurine modification rate of mitochondrial tRNA^{Leu(UUR)}, particularly as a pharmaceutical product, quasi-drug, cosmetic or food for improving the taurine modification rate of normal mitochondrial tRNA^{Leu(UUR)} that decreased due to aging.

The improving agent of the present invention contains taurine as an active ingredient. Taurine is also referred to as aminoethylsulfonic acid or 2-aminoethanesulfonic acid. Taurine may be organically synthesized according to a conventional method or may be a natural product extracted and isolated from animals and plants.

Taurine may be in the form of a taurine derivative as long as it acts as taurine in vivo. Examples of the taurine derivative include N-methyltaurine, N,N-dimethyltaurine, N,N,N-trimethyltaurine, guanidinoethanesulfonic acid, guanidinoethanesulfinic acid, N-(2-acetamide)-2-aminoethanesulfonic acid, piperazino-N,N'-bis(2-ethanesulfonic acid), N-[1'-aza-cycloheptane-2'-yl]-2-aminoethanesulfonic acid, N-[1'-aza-cyclopentane-2'-yl]-2-aminoethanesulfonic acid, N-[1'-aza-cycloheptane-2'-yl]-3-aminopropanesulfonic acid, N-[1'-aza-cyclopentane-2'-yl]-3-aminopropanesulfonic acid, 2-aminoethylphosphonic acid, 3-aminopropionic acid, ethanolamine-O-sulfate, aminomethanesulfonic acid, homotaurine, pyridine-3-sulfonic acid, piperidine-3-sulfonic acid, aniline-2-sulfonic acid, (±)-2-aminocyclohexanesulfonic acid, 2-aminocyclopentanesulfonic acid, quinoline-8-sulfonic acid, 1,2,3,4-tetrahydroquinoline-8-sulfonic acid, 3-amino-bicyclo[2.2.1]heptane-2-sulfonic acid, 6-aminomethyl-3-methyl-4-1,2,4-benzothiadiazine-, 1-dioxide (TAG), glycine, retinylidene taurine(TAURET)3-acetamide-1-propanesulfonic acid Ca salt (ACAMPROSATE), 5-taurinomethyluridine, 5-taurinomethyl-2-thiouridine, isethionic acid, cysteinesulfonic acid, ritralon, 2-amino-3-hydroxy-1-propanesulfonic acid, N-(2,3-dihydroxy-n-propyl)taurine, decanoylsarcosyl taurine and the like.

The taurine modification rate in a test subject is improved by administration or ingestion of the improving agent of the present invention to or by the test subject, as compared to before administration or ingestion. The taurine modification rate to be improved here is a total taurine modification rate represented by the aforementioned formula (1) and/or a normal taurine modification rate represented by the aforementioned formula (2).

For such object, the improving agent of the present invention contains taurine alone, or also contains excipient (e.g., lactose, sucrose, starch, cyclodextrin etc.) and, in some cases, flavor, dye, seasoning, stabilizer, preservative and the like. It is formulated as tablet, pill, granule, fine granule, powder, pellet, capsule, solution, milky lotion, suspension, syrup, lozenge and the like and can be used as medicament, quasi-drug, cosmetic or food (preferably, food with health claims) or food additive. The improving agent of the present invention can also be used as a research reagent.

The amount of taurine contained in the improving agent of the present invention is not particularly limited as long as it affords the effect of the present invention, and is generally 0.0001 - 100 wt%, preferably 0.001 - 99.0 wt%.

The effective amount of taurine can be shown as a daily amount of taurine per 1 kg body weight. A recommended effective amount is 0.01 - 1.0 g/kg body weight/day, preferably 0.02 - 0.5 g/kg body weight/day.

When the improving agent of the present invention is used as a medicament, it preferably contains an effective amount of taurine and a pharmaceutically acceptable carrier.

Examples of the pharmaceutically acceptable carrier include, but are not limited to, excipient (e.g., lactose, sucrose, dextrin, hydroxypropylcellulose, polyvinylpyrrolidone, light anhydrous silicic acid etc.), disintegrant (e.g., starch, carboxymethylcellulose etc.), lubricant (e.g., magnesium stearate, talc etc.), surfactant (e.g., polyoxyethylene hydrogenated castor oil, glycerol monostearate, sodium lauryl sulfate, macrogols, sucrose fatty acid ester etc.), solvent (e.g., water, brine, soybean oil etc.), preservative (e.g., p-hydroxybenzoic acid ester etc.) and the like.

The improving agent of the present invention can be safely administered orally or parenterally to an animal (e.g., mammal, birds, fish etc.).

The improving agent of the present invention can also be ingested as a food or food additive. The "food" in the present invention means food in general, which includes general food including what is called health food, as well as food with health claims such as food for specified health use and food with nutrient function claims, specified in the Food with health claims system by the Japanese Ministry of Health, Labour and Welfare, which may be a food with health claim or health highlighting. The food of the present invention further encompasses supplements, feed/prey and the like.

When used for foods, taurine can also be used by adding to, for example, general foods (including so-called health foods) such as beverage, bread, confectionery and the like. In addition, taurine is formulated together with excipient (e.g., lactose, sucrose, starch etc.) and, in some cases, flavor, dye and the like into tablet, pill, granule, fine granule, powder, pellet, capsule, solution, milky lotion, suspension, syrup, lozenge and the like and can be used as food with health claims such as food for specified health uses, food with nutrient function claims and the like, or supplement. In addition, the food and food additive of the present invention can also be applied to feed/prey use, and can be given or administered to poultry, domestic animals and the like by adding to general feed/prey.

When ingested as a food or feed/prey, general number of ingestion times of food or feed/prey per day and general amount of ingestion for one time are calculated, the daily intake is defined and the amount of taurine contained in the daily intake amount of the food or feed/prey is determined. The content of taurine can be determined based on the below-mentioned doses.

The ingestion or administration amount of the improving agent of the present invention varies depending on the age, body weight and health condition of the subject of ingestion or administration and cannot be determined unconditionally. For example, when aiming at maintaining, promoting or improving health, the agent is generally formulated in the form of a quasi-drug such as drink agent and the like, food or cosmetic. When treatment of disorders caused by a decrease in the taurine modification rate or health recovery is aimed, the agent is generally formulated in the form of a pharmaceutical product, quasi-drug or food. It is preferable to give or administer 0.4 - 40 g, preferably 0.8 g - 20 g, more preferably 4.0 - 15 g, as taurine per day for an adult in one to several portions per day. When the body weight is 40kg or below, it is preferable to generally give or administer 0.3 - 30 g, preferably 0.6 g - 15 g, more preferably 3.0 - 12 g, per day in one to several portions per day.

The administration method of the improving agent (medicament) of the present invention is not particularly limited as long as it is a route affording a prophylactic or therapeutic effect on a decrease in the taurine modification rate. For example, the agent can be administered parenterally (intravenous administration, intramuscular administration, direct administration into tissues, intranasal administration, intradermal administration, transdermal administration, intraperitoneal administration, gastrostomy, tube administration, enteral nutrition administration and the like) or orally. Particularly, when the medicament is applied to human, it can be administered intravenously, intramuscularly or orally. The dosage form is not particularly limited, and various administration dosage forms, for example, oral preparation (granule, powder, tablet, capsule, syrup, emulsion, suspension, drink agent and the like), injection, drip infusion, external preparation (preparations for nasal administration, dermal preparation, ointment and the like) can be used for administration. An external preparation (dermal preparation, ointment and the like) can also be administered as a quasi-drug or cosmetic.

An agent for improving taurine modification rate of mitochondrial tRNA^{Leu(UUR)} of the present invention may be used as an agent for improving the taurine modification rate of mitochondrial tRNA^{Leu(UUR)} that has decreased due to aging and the state of biological function due to genetic factors and lifestyle and environmental factors, and the like, in patients who developed mitochondrial diseases, preferably, patients who developed mitochondrial diseases due to the mutation of a gene deficient in taurine modification of mitochondrial tRNA^{Leu(UUR)}, more preferably patients who developed mitochondrial diseases due to a point mutation selected from the group consisting of the 14-position A (A3243G), the 15-position G (G3244A), the 29-position T (T3258C), the 42-position T (T3271C) and the 62-position T (T3291C) in a DNA shown in SEQ ID NO: 1 and encoding tRNA^{Leu(UUR)}, particularly preferably patients who developed mitochondrial diseases due to a point mutation at the 14-position A (A3243G) in a DNA shown in SEQ ID NO: 1 and encoding tRNA^{Leu(UUR)}, particularly, patients who developed MELAS or diabetes or a subject having a risk thereof. Furthermore, the improving agent of the present invention may also be used as an agent for improving the taurine modification rate of mitochondrial tRNA^{Leu(UUR)} that has decreased due to aging and the state of biological function due to genetic factors, lifestyle and environmental factors, and the like, in these targets and patients. In addition, the agent for improving taurine modification rate of mitochondrial tRNA^{Leu(UUR)} of the present invention is particularly useful not only for the improvement of abnormal taurine modification rate of mitochondrial tRNA^{Leu(UUR)}, but also for the improvement of a decrease in the taurine modification rate of normal mitochondrial tRNA^{Leu(UUR)}.

### [Examples]

The present invention is explained in more detail in the following by referring to Examples. The present invention is not limited thereto in any way.

### Blood sample

A written informed consent was obtained from MELAS patients who participated in the clinical trial and patient blood was collected. Crude RNA was obtained from the collected blood by using Isogen (manufactured by Nippon Gene) according to the manufacturer's instructions.

Handling of patients' blood also followed the ethical regulations at Kawasaki Medical School.

### Example 1 Detection of taurine modification of mitochondrial tRNA^{Leu(UUR)} by reverse transcription reaction

Basically, the method of Kirino et al. (Proc Natl Acad Sci U S A. 2005; 102(20): 7127-7132) was followed. The principle of the primer elongation method by Kirino et al. is shown in Fig. 1.

The base sequence of the primer used is as described below.
5'-acctctgactgtaaag-3' (SEQ ID NO: 3)

The base sequences of the two predicted primer elongation products are as follows.
5'-acctctgactgtaaagTTTTAAG-3' (SEQ ID NO: 6: sequence elongated from the primer is indicated by capital letters)
5'-acctctgactgtaaagTTTTAG-3' (SEQ ID NO: 7: sequence elongated from the primer is indicated by capital letters)

The 5'-terminal of the above-mentioned primer and primer elongation product is labeled with ³²P in this Example.

### (reverse transcription reaction)

Using RNA (0.2 - 1 µg) obtained from the leukocyte of the test subject as a template, the primer was annealed by incubating with ³²P-labeled primer (SEQ ID NO: 3, 0.1 pmol) corresponding to the 3' side of the anticodon of tRNA^{Leu(UUR)} at 80°C for 2 min, and standing the mixture at room temperature for 1 hr. Thereafter, in a reaction buffer for reverse transcriptase (manufactured by Invitrogen), dATP, dTTP, ddGTP (each 1.5 mM, manufactured by AmershamPharmacia) were added (final concentration 37.5 µM), M-MuLV Reverse transcriptase (RNase H⁻) 1 µL (40 units/pL, manufactured by Invitrogen) was mixed and a reverse transcription reaction was performed at 42°C for 1 hr. In the case of MELAS patients having T3271C mutation, ³²P-labeled primers (mix primer, 0.1 pmol) shown in SEQ ID NO: 3 and SEQ ID NO: 4 were used.

After completion of the reaction, the reaction mixture was subjected to 15% polyacrylamide electrophoresis containing 7M urea. The band labeled with RI was visualized with BAS5000 bio-imaging analyzer (manufactured by Fuji Film).

When the anticodon (first letter U: indicated by underline in Fig. 1) lacks taurine modification (⁵U₃₄), the reaction reaches AAG and when taurine modification is present (τm⁵U₃₄), the reaction stops at AG. The RI-labeled reaction product was separated by electrophoresis, quantified by BAS5000 Bio-imaging analyzer (manufactured by Fuji Film), and total taurine modification rate was determined. The results are shown in Fig. 2.

Since MELAS patients who participated in doctor-initiated clinical trials had A3243G mutation or T3271C mutation, these point mutation rates were determined as follows. A partially-modified PCR-RFLP method of Goto et al. was used (Goto Y. et al.: A new mtDNA mutation associated with mitochondrial myopathy, encephalopathy, lactic acidosis and stroke-like episodes (MELAS). Biochimica et Biophysica Acta 1097: 238-240 (1991)). For detection of A3243G mutation, PCR was performed using two primers of
For: 5'-gcccttcccccgtaaatgatat-3' (SEQ ID NO: 8) and
Rev: 5'-gaagaggaattgaacctctgactg-3' (SEQ ID NO: 9), and the amplified PCR product was cleaved with restriction enzyme ApaI. Normal mtDNA does not have a cleavage site of restriction enzyme ApaI, and a single band of 136 bp is obtained. In A3243G mutation, since there is a cleavage site of ApaI, two bands of 84 bp and 52 bp appear by cleavage. For T3271C mutation, PCR was performed using two primers of
For: 5'-taagaagaggaattgaacctctgaccttaa-3' (SEQ ID NO: 10) and
Rev: 5'-aggacaagagaaataaggcc-3' (SEQ ID NO: 11), and the amplified PCR product was cleaved with restriction enzyme AflII. Normal mtDNA does not have a cleavage site of restriction enzyme AflII, and a single band of 170 bp is obtained. In T3271C mutation, since there is a cleavage site of AflII, two bands of 140 bp and 30 bp appear by cleavage.

The PCR product cleaved with the above-mentioned restriction enzyme was subjected to 15% polyacrylamide electrophoresis, the separated band was stained with ethidium bromide, the intensity of the band was quantified using a densitometer, and the point mutation rate was determined (Table 1) .

Using the above-mentioned total taurine modification rate and point mutation rate, the normal taurine modification rate of MELAS patients was determined. The results are shown in Fig. 2.

### Test Example 1 Measurement of lactic acid value and pyruvic acid value of blood and cerebrospinal fluid

Measurement of the lactic acid value in the blood and in the cerebrospinal fluid was performed by reacting lactic acid oxidase with L-lactic acid and quantifying the resulting hydrogen peroxide by colorimetrically measuring the dye generated in the presence of peroxidase.

The pyruvic acid value was determined by reacting pyruvic acid oxidase with pyruvic acid and measuring the generated hydrogen peroxide by colorimetrically measuring the dye generated in the presence of peroxidase.

### Results

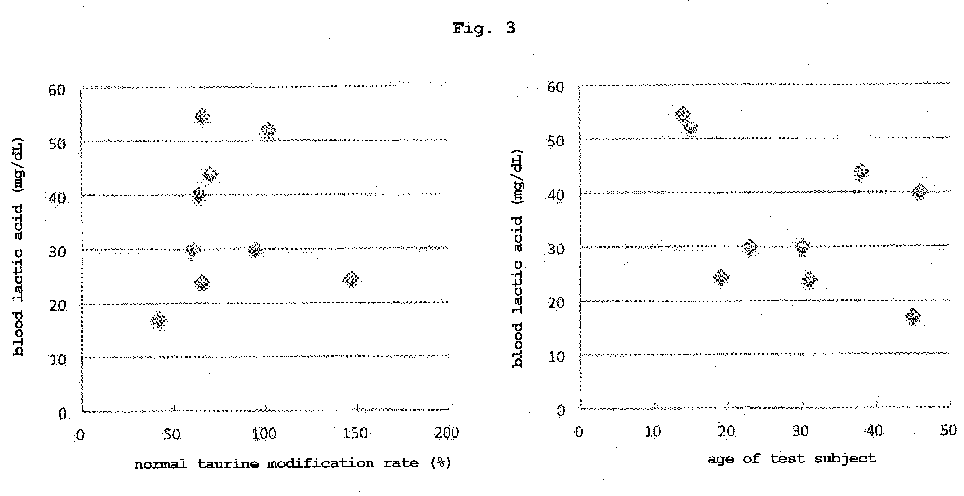

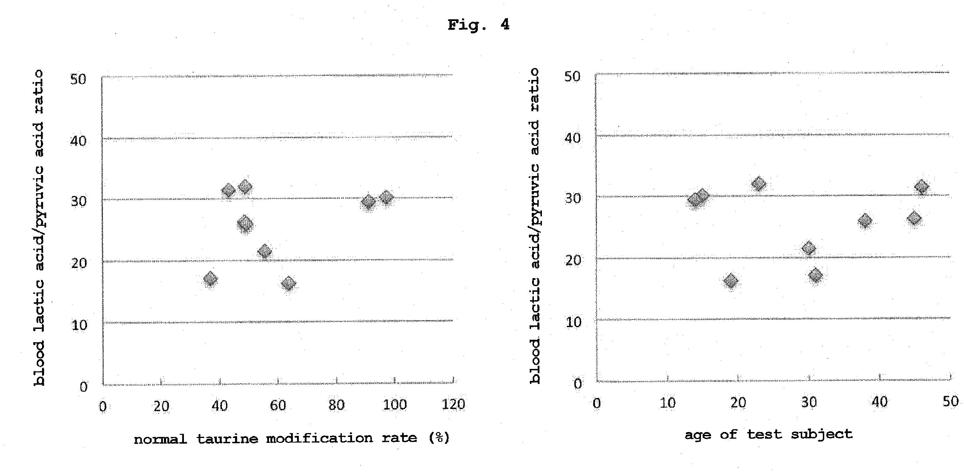

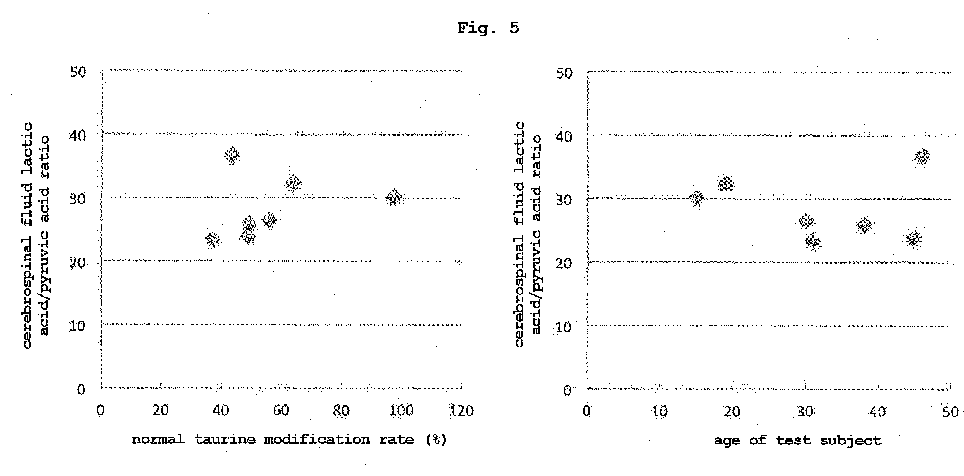

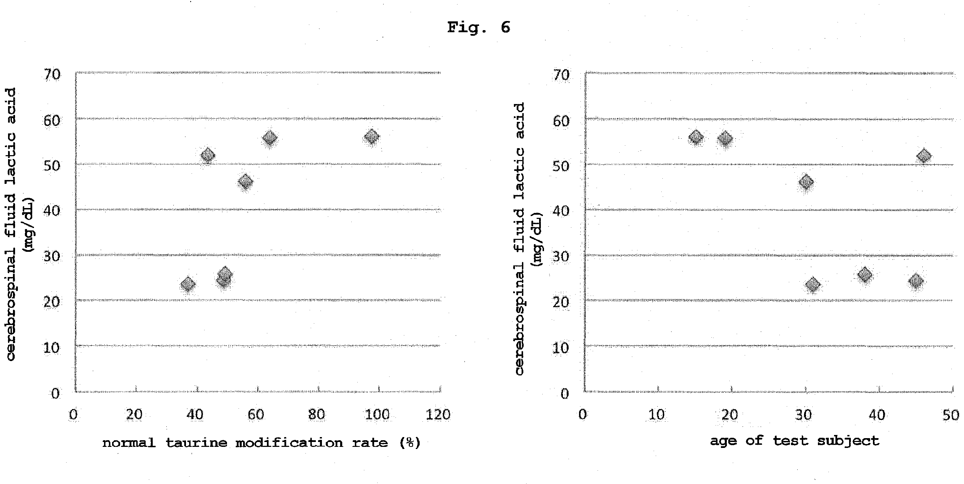

Taurine modification rate of mitochondrial tRNA^{Leu(UUR)} in blood leukocytes of patients participating in the clinical trial was measured and show graphically with the age of the test subject plotted as the horizontal axis. As a result, the total taurine modification rate and the age were not correlated, whereas the normal taurine modification rate decreased in an age-dependent manner (Fig. 2). On the other hand, the normal taurine modification rate did not correlate with blood and cerebrospinal fluid lactic acid values, lactic acid/pyruvic acid ratio, which are conventional mitochondrial disease markers, and did not correlate with the age of the test subject and conventional mitochondrial disease marker (Figs. 3-6).

### Test Example 2 Improvement of taurine modification rate by administration of taurine to MELAS patients

In the above-mentioned doctor-initiated clinical trial, taurine was administered to MELAS patients. The dose was 12 g/day for test subjects weighing not less than 40 kg, 9 g/day for not less than 25 kg to less than 40 kg, and the dosing period was 52 weeks (1 year). Blood was collected from the test subject at the end date of the taurine administration (52 weeks), and the total taurine modification rate and the normal taurine modification rate were measured according to the method described in Example 1. The results are shown in Fig. 7 together with the data on the administration start date (0 week before start) obtained in Example 1. In addition, the total taurine modification rate, normal taurine modification rate and point mutation rate of each MELAS patient on the start date of taurine administration and after completion of the administration are collectively shown in Table 1. The total taurine modification rate was compared between before and after taurine administration to find tendency toward a significant difference (P=0.0546). It was clarified that the normal taurine modification rate was recovered and the taurine modification of tRNA^{Leu(UUR)} derived from mutated mtDNA was improved by taurine ingestion.

**[Table 1]**

| age of test subject | before administration (week 0) | | | after administration (week 52) | | |
|---|---|---|---|---|---|---|
| | mtDNA point mutation rate (%) | total taurine modification rate (%) | normal taurine modification rate (%) | mtDNA point mutation rate (%) | total taurine modification rate (%) | normal taurine modification rate (%) |
| 14 | 57.8 | 38.5 | 91.2 | 56.0 | 28.4 | 65.8 |
| 15 | 65.8 | 33.3 | 97.4 | 66.2 | 34.5 | 102.1 |
| 19 | 53.0 | 30.0 | 63.7 | 54.3 | 67.2 | 147.0 |
| 23 | 39.4 | 29.7 | 48.9 | 138.6 | 58.1 | 94.7 |
| 30 | 43.4 | 31.5 | 55.7 | 44.0 | 33.7 | 60.3 |
| 31 | 30.9 | 25.5 | 37.0 | 29.2 | 46.4 | 65.6 |
| 38 | 21.5 | 38.5 | 49.1 | 19.1 | 56.9 | 70.3 |
| 45 | 29.5 | 34.3 | 48.6 | 26.7 | 30.7 | 41.8 |
| 46 | 28.7 | 31.0 | 43.4 | 33.8 | 42.3 | 63.9 |

The results of Example 1 and Test Example 2 show that the taurine modification rate of normal mitochondrial tRNA^{Leu(UUR)} decreases with aging and that the decreased taurine modification rate increases by oral administration of taurine. Furthermore, the presence of cases in which the taurine modification rate of normal tRNA^{Leu(UUR)} exceeds 100% indicates that the taurine modification rate of mutated tRNA^{Leu(UUR)} is also improved by oral administration of taurine.

The cause of the onset of MELAS has heretofore been considered to be the lack of taurine modification of mutated tRNA^{Leu(UUR)}; however, this result suggests that the onset of MELAS involves a decrease in the taurine modification rate of normal tRNA^{Leu(UUR)} due to aging.

As is well known, taurine modification function of tRNA^{Leu(UUR)} itself is not deficient in MELAS patients.

While the phenomenon observed in Example 1 is the analysis result of MELAS patients, it can be said to reflect a phenomenon that occurs similarly in healthy subjects as well. That is, the taurine modification rate of tRNA^{Leu(UUR)} can be used as a biomarker of mitochondrial senescence.

When the taurine modification rate of mitochondrial tRNA^{Leu(UUR)} decreases along with senescence, the UUG codon-specific translation ability naturally becomes rate-limiting and synthesis of the whole protein including the UUG codon decreases. It is obvious that, along with that, the mitochondrial function that provides an important function in respiration and energy metabolism declines, and the functions of the living body, the organs constituting the living body and the like deteriorate. If the decline of the modification rate progresses, for example, serious symptoms such as dyspnea and attenuation of skeletal muscle found in elderly people are considered to be developed in some cases.

Therefore, the results of Example 1 and Test Examples show that the taurine modification rate of normal mitochondrial tRNA^{Leu(UUR)} is also useful as an index of the functions of living organisms, organs, tissues, cells or intracellular organella, in particular, as an index of senescence and functional deterioration of living organisms and organs, tissues, cells or intracellular organelle having high energy demand, not to mention that it can be used as an index of senescence and functional deterioration of mitochondrion itself. In addition, cases with lower normal taurine modification rate than that corresponding to the chronological age as in Test Example 2 with a patient having a test subject age of 14 and cases with relatively high normal taurine modification rate despite a relatively high chronological age as in the patient having a test subject age of 46 are observed, and there is a considerable range in the normal taurine modification rate of the young healthy subjects of the below-mentioned Test Example 4. Therefore, it is shown that the normal taurine modification rate is not only a mere index of age-related senescence of a test subject but also utilizable as an index of senescence reflecting the health condition of a test subject including nutritional state, the presence or absence of a disease such as metabolic abnormality and the like, and the like, or as an index of health expectancy, in particular, health expectancy without any specific cause of death.

Furthermore, a decrease in the taurine modification rate of normal mitochondrial tRNA^{Leu(UUR)}, which is associated with senescence due to aging and the like, can be improved by administration of taurine. Thus, the results of Test Example 2 and the below-mentioned Test Example 3 show that taurine and a composition containing same are useful as pharmaceutical products, quasi-drugs, cosmetics or foods for improving the taurine modification rate of mitochondrial tRNA^{Leu(UUR)}, preventing a decrease in the taurine modification rate of mitochondrial tRNA^{Leu(UUR)}, in particular, improving taurine modification rate of normal mitochondrial tRNA^{Leu(UUR)} that decreased due to aging.

### Test Example 3 Improvement of diabetes marker by administration of taurine to diabetes patients

In Test Example 2, the disease state was improved by taurine administration in MELAS patients due to single base mutation (A3243G) of mitochondrial gene. Thus, the treatment effect of taurine administration on diabetes caused by the same single base mutation (A3243G) of mitochondrial gene was confirmed. To be specific, taurine (trade name taurine powder 98% "Taisho", manufactured by Taisho Pharmaceutical Co. Ltd.) was orally administered every day to a patient (49 years old, female) diagnosed to have diabetes due to single base mutation (A3243G) of mitochondrial gene and having cardiomyopathy in association therewith. Administered was taurine at 4 g/administration, 3 times/day (12 g/day), and the dosing period was about one year. Blood was collected from this patient over time from the start to the end of taurine administration, and the value of hemoglobin A1c (HbA1c), which is a diabetic marker, was measured. As a result, the value of 7.7% at the start decreased to 6.9% at week 23 (about 5 months) from the start of administration and 6.8% at the end of administration. As the above-mentioned results show, the value of HbAlc of this patient was controlled to less than 7%, which is the desired value for preventing complications of diabetes, throughout the taurine administration period. Furthermore, 12 units of insulin administration necessary at the start of taurine administration could be reduced to 9 units at the end of administration, and it was confirmed that oral administration of taurine can improve the symptoms of diabetes patients due to mitochondrial gene mutation.

As is clear from the below-mentioned Test Example 4, a decrease in the taurine modification rate of normal mitochondrial tRNA^{Leu(UUR)} caused by aging and the state of biological function due to genetic factors, lifestyle and environmental factors, and the like is not limited to MELAS but is a universal phenomenon in healthy subjects as well. Thus, the mechanism of the onset of diabetes due to mitochondrial gene mutation is considered to be based on a decrease in the taurine modification rate of normal mitochondrial tRNA^{Leu(UUR)} in addition to abnormal mitochondrial tRNA^{Leu(UUR)}, similar to the case of MELAS. Therefore, also in the case of diabetes, the clinical symptoms can be considered to have been improved by the improvement of taurine modification rate of normal mitochondrial tRNA^{Leu(UUR)} in addition to the improvement of taurine modification rate of abnormal mitochondrial tRNA^{Leu(UUR)} by taurine administration.

### Test Example 4 Measurement of taurine modification rate of normal mitochondrial tRNA^{Leu(UUR)} in healthy subjects

In Test Example 2, it was confirmed that the taurine modification rate of normal mitochondrial tRNA^{Leu(UUR)} decreases with aging in MELAS patients. Thus, it was confirmed that the taurine modification rate of normal mitochondrial tRNA^{Leu(UUR)} similarly decreases with aging in healthy subjects as well by a method similar to that in Test Example 2. The results thereof are shown in Fig. 8. In the analysis following the method described in Example 1 and using peripheral blood leukocyte samples of three young healthy subjects (10's) and three elderly healthy subjects (80's), the modification rate of normal mitochondrial tRNA^{Leu(UUR)} was 35.8±3.0% in elderly people (80's) as compared to average 56.7±12.6% (Mean±SE) of young people (10's), and a significant decrease due to aging was observed. When compared among young people, the modification rate ranges about twice in width from about 80% to about 40%. Thus, it is considered that the decrease in the taurine modification rate of normal mitochondrial tRNA^{Leu(UUR)} reflects not only aging but also decline in the biological functions due to genetic factors, lifestyle and environmental factors, and the like. The results indicate that the taurine modification rate of normal mitochondrial tRNA^{Leu(UUR)} can be used as a useful biomarker of senescence not only in MELAS patients but also in healthy subjects.

The above-mentioned Test Examples show that the taurine modification rate of normal mitochondrial tRNA^{Leu(UUR)} universally decreases with senescence due to aging or by an influence of genetic factors, lifestyle and environmental factors and the like, both in mitochondrial disease patients and healthy subjects.

In addition, it was confirmed that a decrease in the aforementioned taurine modification rate is improved by taurine administration. Thus, the agent for improving the taurine modification of mitochondrial tRNA^{Leu(UUR)} of the present invention is useful for improving or preventing a decrease in the taurine modification rate of mitochondrial tRNA^{Leu(UUR)}, particularly, a decrease in the taurine modification rate of normal mitochondrial tRNA^{Leu(UUR)}, associated with senescence, in, in addition to healthy subjects, non-disease subjects and subjects who are at risk of developing some disease, as well as patients having some disease, in particular, mitochondrial disease patients and subjects at risk of developing such disease.

Furthermore, the taurine modification rate of normal mitochondrial tRNA^{Leu(UUR)} is useful as a biomarker for selection of subjects of taurine therapy currently under development for mitochondrial disease patients, use for companion diagnosis to be used for determination of the treatment effect thereof and assistance therefor.

### Formulation Example 1 Granule (in one packet)

| | |
|---|---|
| taurine | 3000 mg |
| aspartame | 9 mg |
| talc | 20 mg |
| low substituted hydroxypropylcellulose | 60 mg |

A granule of the above-mentioned formulation was prepared by a conventional method.

### Formulation Example 2 Internal liquid agent (in one agent)

| | |
|---|---|
| Cinnamomi Cortex | 100 mg |
| royal jelly | 100 mg |
| taurine | 5000 mg |
| 70% sorbitol solution | 1000 mg |
| erythritol | 1000 mg |
| pH adjuster | q.s. |

Purified water is added to a total amount of 180 mL.

An internal liquid of the above-mentioned formulation was prepared by a conventional method.

### Formulation Example 3 Drink agent (in one agent)

| | |
|---|---|
| vitamin B1 nitrate salt | 10 mg |
| vitamin B2 | 2 mg |
| carnitine chloride | 50 mg |
| taurine | 4000 mg |
| pantothenic acid sodium | 10 mg |
| benzoic acid | 20 mg |
| butyl paraoxybenzoate | 2.5 mg |
| aspartame | 6 mg |
| citric acid | 100 mg |
| glycine | 300 mg |

Purified water is added to a total amount of 50 mL.

A drink agent of the above-mentioned formulation was prepared by a conventional method.

### [Industrial Applicability]

The present inventors have found that the taurine modification rate of normal tRNA^{Leu(UUR)}, among the mitochondrial tRNAs, serves as an index of senescence. The taurine modification rate of normal tRNA^{Leu(UUR)} is a universal marker capable of grasping the state of not only mitochondrial disease patients but also healthy individuals. Taurine is useful as a pharmaceutical product, quasi-drug, cosmetic or food for improving the taurine modification rate of mitochondrial tRNA^{Leu(UUR)} that decreased due to aging and an influence of, for example, lifestyle and environmental factors such as intense fatigue and the like, genetic factor and the like, and the like.

While some of the embodiments of the present invention have been described in detail in the above, those of ordinary skill in the art can make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2016-062910 filed in Japan (filing date: March 26, 2016), the contents of which are incorporated in full herein.

## Claims

1. A method for measuring senescence of a test subject, comprising a step of measuring a level of taurine modification of mitochondrial tRNA^{Leu(UUR)} in a biological sample isolated from a test subject, by a reverse transcription reaction from a primer with the tRNA^{Leu(UUR)} as a template.

2. The method according to claim 1, wherein the primer is an oligonucleotide 10 - 25 bases in length and complementary to the template, and a difference in primer elongation products is detected based on the presence or absence of taurine modification.

3. The method according to claim 1 or 2, wherein the primer is at least one kind comprising a base sequence selected from the group consisting of base sequences shown in SEQ ID NOs: 3, 4 and 5.

4. The method according to any one of claims 1 to 3, further comprising a step of calculating a quantitative ratio of a primer elongation product from tRNA^{Leu(UUR)} with taurine modification and a primer elongation product from tRNA^{Leu(UUR)} without taurine modification.

5. The method according to any one of claims 1 to 4, wherein a total taurine modification rate represented by the following formula (1): $total taurine modification rate = τ {m}^{5} U / \left(U + τ {m}^{5} U\right) x 100 \left(%\right)$ wherein τm⁵U is an amount of primer elongation product from tRNA^{Leu(UUR)} with taurine modification and U is an amount of a primer elongation product from tRNA^{Leu(UUR)} without taurine modification, is an index of senescence.

6. The method according to claim 5, wherein the test subject is suspected to have a mutated mitochondrial DNA having a single base substitution selected from the group consisting of A3243G, T3271C, G3244A, T3258C and T3291C in the tRNA^{Leu(UUR)} gene coding region, and a normal taurine modification rate determined by the following formula (2): $normal taurine modification rate = total taurine modification rate / \left(100 - mtDNA point mutation rate\right) / 100 \left(%\right)$ (in the above-mentioned formula, the mtDNA point mutation rate is the highest point mutation rate selected from the group consisting of point mutations at 14-position A (A3243G), 15-position G (G3244A), 29-position T (T3258C), 42-position T (T3271C) and 62-position T (T3291C) in a DNA encoding tRNA^{Leu(UUR)} shown in SEQ ID NO: 1) successively from the aforementioned formula (1) is the index of senescence.

7. A senescence determining drug comprising a primer comprising a base sequence consisting of 10 - 25 bases and complementary to the mitochondrial tRNA^{Leu(UUR)}.

8. The drug according to claim 7, wherein the primer is at least one kind comprising a base sequence selected from the group consisting of the base sequences shown in SEQ ID NOs: 3, 4 and 5.

9. A senescence determination kit comprising a primer comprising a base sequence consisting of 10 - 25 bases and complementary to the mitochondrial tRNA^{Leu(UUR)} and a reverse transcriptase.

10. The kit according to claim 9, wherein the primer is at least one kind comprising a base sequence selected from the group consisting of the base sequences shown in SEQ ID NOs: 3, 4 and 5.

11. An agent for improving a taurine modification rate of mitochondrial tRNA^{Leu(UUR)}, comprising taurine as an active ingredient.

12. The agent according to claim 11, wherein the mitochondrial tRNA^{Leu(UUR)} is normal mitochondrial tRNA^{Leu(UUR)}.

13. The agent according to claim 11, wherein the improvement of taurine modification rate is improvement of a total taurine modification rate determined from the following formula (1): $total taurine modification rate = τ {m}^{5} U / \left(U + τ {m}^{5} U\right) x 100 \left(%\right)$ wherein τm⁵U is an amount of a primer elongation product from tRNA^{Leu(UUR)} with taurine modification and U is an amount of a primer elongation product from tRNA^{Leu(UUR)} without taurine modification, and/or a normal taurine modification rate determined from the following formula (2): $normal taurine modification rate = total taurine modification rate / \left(100 - mtDNA point mutation rate\right) / 100 \left(%\right)$ (in the above-mentioned formula, the mtDNA point mutation rate is the highest point mutation rate selected from the group consisting of point mutations at 14-position A (A3243G), 15-position G (G3244A), 29-position T (T3258C), 42-position T (T3271C) and 62-position T (T3291C) in a DNA encoding tRNA^{Leu(UUR)} shown in SEQ ID NO: 1).

14. The agent according to claim 11 or 12, which is a medicament or cosmetic.

15. The agent according to claim 11 or 12, which is a food with health claims or food additive.

16. The agent according to any one of claims 11 to 15, which is for improving a taurine modification rate of mitochondrial tRNA^{Leu(UUR)} of a patient who developed a mitochondrial disease and/or a subject having an onset risk.

17. The agent according to claim 16, wherein the mitochondrial disease is caused by a mutation of a gene deficient in taurine modification of mitochondrial tRNA^{Leu(UUR)}.

18. The agent according to claim 17, wherein the mutation of a gene deficient in taurine modification of mitochondrial tRNA^{Leu(UUR)} is a point mutation selected from the group consisting of the 14-position A (A3243G), the 15-position G (G3244A), the 29-position T (T3258C), the 42-position T (T3271C) and the 62-position T (T3291C) in a DNA encoding tRNA^{Leu(UUR)} shown in SEQ ID NO: 1).

19. The agent according to any one of claims 16 to 18, wherein the mitochondrial disease is MELAS or diabetes.


SEQUENCE LISTING

<110>  Kawasaki Gakuen Educational Foundation

<120>  Mitochondrial biomarker that reflects senescence

<130>  092598

<150>  JP 2016-062910
<151>  2016-03-26

<160>  11

<170>  PatentIn version 3.5

<210>  1
<211>  78
<212>  DNA
<213>  Homo sapiens

<400>  1
gttaagatgg cagagcccgg taatcgcata aaacttaaaa ctttacagtc agaggttcaa      60

ttcctcttct taacacca                                                    78


<210>  2
<211>  78
<212>  RNA
<213>  Homo sapiens


<220>
<221>  tRNA
<222>  (1)..(78)

<220>
<221>  modified_base
<222>  (9)..(9)
<223>  m1g

<220>
<221>  modified_base
<222>  (10)..(10)
<223>  m2g

<220>
<221>  modified_base
<222>  (21)..(21)
<223>  dhu

<220>
<221>  modified_base
<222>  (29)..(29)
<223>  p

<220>
<221>  modified_base
<222>  (50)..(50)
<223>  m5c

<220>
<221>  modified_base

<222>  (56)..(56)
<223>  t

<220>
<221>  modified_base
<222>  (57)..(57)
<223>  p

<220>
<221>  modified_base
<222>  (60)..(60)
<223>  m1a

<400>  2
guuaagaugg cagagcccgg uaaucgcaua aaacuuaaaa cuuuacaguc agagguucaa      60

uuccucuucu uaacacca                                                    78


<210>  3
<211>  16
<212>  DNA
<213>  Artificial Sequence

<220>
<223>  primer

<400>  3
acctctgact gtaaag                                                      16


<210>  4
<211>  16
<212>  DNA
<213>  Artificial Sequence

<220>
<223>  primer

<400>  4
acctctgact gtaagg                                                      16


<210>  5
<211>  16
<212>  DNA
<213>  Artificial Sequence

<220>
<223>  primer

<400>  5
acctctgact gcaaag                                                      16


<210>  6
<211>  23
<212>  DNA
<213>  Artificial Sequence

<220>
<223>  extension product

<400>  6
acctctgact gtaaagtttt aag                                              23


<210>  7
<211>  22
<212>  DNA
<213>  Artificial Sequence

<220>
<223>  extension product

<400>  7
acctctgact gtaaagtttt ag                                               22


<210>  8
<211>  22
<212>  DNA
<213>  Artificial Sequence

<220>
<223>  forward primer

<400>  8
gcccttcccc cgtaaatgat at                                               22


<210>  9
<211>  24
<212>  DNA
<213>  Artificial Sequence

<220>
<223>  Reverse primer

<400>  9
gaagaggaat tgaacctctg actg                                             24


<210>  10
<211>  30
<212>  DNA
<213>  Artificial Sequence

<220>
<223>  Forward primer

<400>  10
taagaagagg aattgaacct ctgaccttaa                                       30


<210>  11
<211>  20
<212>  DNA
<213>  Artificial Sequence

<220>
<223>  Reverse primer

<400>  11
aggacaagag aaataaggcc                                                  20